# EUROPÄISCHE PATENTSCHRIFT

(11) **EP 2 223 715 B1**
(45) Veröffentlichungstag und Bekanntmachung des Hinweises auf die Patenterteilung: **12.04.2017**
(21) Anmeldenummer: 09180471.6
(22) Anmeldetag: 22.12.2009
(51) Int. Cl.: A61M 5/31, C03B 9/193

(54) **Herstellung eines pharmazeutischen Behälters aus der Schmelze**
Production of a pharmaceutical container from a molten mass
Fabrication d'un récipient pharmaceutique à partir de la masse fondue

(30) Priorität: 06.02.2009 DE 102009008723
(43) Veröffentlichungstag der Anmeldung: 01.09.2010
(73) Patentinhaber: Schott AG, 55122 Mainz (DE)
(72) Erfinder: Langsdorf, Andreas, 55218, Ingelheim (DE); Thürk, Jürgen, 9008, St. Gallen (CH); Kunz, Aurel, 9050, Appenzell (CH); Lange, Ulrich, 55122, Mainz (DE); Küster, Joachim, 63490, Erzhausen (DE); Löffelbein, Bernd, 55291, Saulheim (DE); Meinefeld, Marcus, 31863 Coppenbruegge (DE); Rothhaar, Uwe, 67134, Birkenheide (DE); Ohlinger, Axel, 65205, Wiesbaden (DE)
(74) Vertreter: Witte, Weller & Partner Patentanwälte mbB

(56) Entgegenhaltungen:
- EP-A- 0 139 339
- DE-B- 1 241 057
- DE-C1- 19 812 056
- GB-A- 572 984
- US-A- 5 540 666
- US-A1- 2006 039 080

## Beschreibung

Die Erfindung betrifft ein Verfahren zum Herstellen eines Behälters in Form eines an beiden Enden offenen Hohlkörpers für pharmazeutische oder medizinische Anwendungen aus Glas, insbesondere in Form eines Spritzenkörpers.

Pharmazeutische Behältnisse werden standardmäßig aus Rohren oder über Blas-verfahren direkt aus der Schmelze hergestellt. Aufgrund geringerer Dickenschwankungen können die Behältnisse aus Rohren hierbei dünnwandiger hergestellt und mit einer engen Wanddickentoleranz gefertigt werden. Aufgrund der Wanddickenproblematik werden Behälter in Form von Spritzenkörpern für pharmazeutische oder medizinische Anwendungen ausschließlich aus Rohren hergestellt.

In der GB 572 984 wird zwar vorgeschlagen, eine Spritze mit zumindest eng spezifiziertem Innendurchmesser über Blasen mittels einer Glasbläserpfeife und Aufschrumpfen auf einen Dorn zu formen. Hierbei handelt es sich jedoch um einen kaum industrialisierbaren Prozess, bei dem mehr als 50 % des Glases verlorengehen und verworfen werden müssen.

Aus der DE 1 241 057 A ist ferner eine Herstellung von Ampullen für pharmazeutische und medizinische Zwecke unmittelbar aus der Glasschmelze bekannt, wobei mittels Glasmacherpfeifen zunächst aus der Glasschmelze unmittelbar die benötigte Gasmenge angesaugt wird und anschließend bei rotierender Pfeife in eine von außen angelegte zweiteilige Form geblasen wird. Die Ampulle wird anschließend durch einen Brenner von der Glasmacherpfeife abgetrennt. Eine andere Art der Erzeugung von Ampullen direkt aus dem Glasbad besteht darin, dass der Schmelzbehälter an seinem Boden eine Öffnung besitzt, durch die das flüssige Glas mittels eines Stößels von oben nach unten hindurchgedrückt wird. Der Glastropfen wird durch eine automatisch arbeitende Schere abgeschnitten, fällt nun in eine trichterförmige Vertiefung und wird dort entweder mittels eines Vakuums oder mittels eines darüber greifenden Ringes festgehalten. Ein automatisch eingeführter Hohldorn bläst dann den Külbel zu einem Hohlkörper auf.

Diese und andere Varianten der Herstellung von Ampullen liefern keine zufriedenstellende Dickentoleranz für die Herstellung von Spritzenkörpern.

Im Stand der Technik hat sich daher zur Herstellung von pharmazeutischen Behältern in Form von Spritzenkörpern eine Herstellung aus vorgeformten Glasrohren durchgesetzt.

Die Nachteile dieses Verfahrens liegen in relativ hohen Herstellungskosten, sowie in der Tatsache, dass durch die lokale Wiedererwärmung des Glasrohres bei der Umformung eine Verarmung an bestimmten Komponenten durch Abdampfen auftritt. So weisen aus Glasrohren hergestellte Behälter in der Regel an verschiedenen Oberflächenbereichen eine Verarmung an Bor und an Natrium auf, soweit die Herstellung aus Borosilikatgläsern erfolgt. Außerdem sind aufgrund von Zwischenverpackung und -transport der Ausgangsrohre zur Umfangsstation zusätzliche Maßnahmen zu treffen, um eine nicht gewünschte Verschmutzung oder Beschädigung der Rohre zu verhindern.

Für die Herstellung von pharmazeutischen oder medizinischen Behältern der hier behandelten Art werden in der Regel sog. "Typ-I-Gläser", auch teilweise als "Neutralgläser" bezeichnet, verwendet. Hierbei handelt es sich um Gläser, in der Regel um Borosilikatgläser, die eine Wasserbeständigkeit der Klasse 1 nach DIN ISO 719 und eine Säurebeständigkeit der Klasse 1 nach DIN 12116 aufweisen.

Aus der US 2006/039080 A1 ist schließlich ein Glasabstandsring für eine Magnetplatte bekannt, der hergestellt wird, indem aus einem Glasrohr mit einem Schneider, einer Diamantsäge oder einem Diamantbohrer ein Ring der gewünschten Dicke abgeschnitten wird und anschließend eine elektrisch leitfähige Schicht auf dem Ring abgeschieden wird.

Der Erfindung liegt die Aufgabe zugrunde, ein Verfahren zur Herstellung von an beiden Seiten offenen Behältern für medizinische oder pharmazeutische Zwecke unmittelbar aus der Glasschmelze bereitzustellen, die insbesondere als Spritzenkörper oder Karpulen einsetzbar sind, wobei die notwendigen Toleranzen für derartige Behälter eingehalten werden können.

Diese Aufgabe wird erfindungsgemäß durch ein Press-Blas-Verfahren mit den Merkmalen des Anspruchs 1 gelöst. Die Aufgabe der Erfindung wird auf diese Weise vollkommen gelöst.

Es hat sich nämlich gezeigt, dass mit einem Press-Blas-Verfahren die Herstellung eines Behälters in Form eines an beiden Enden offenen Hohlkörpers für pharmazeutische oder medizinische Anwendungen unmittelbar aus der Glasschmelze mit ausreichender Maßgenauigkeit möglich ist.

Dies hat erhebliche Kostenvorteile, da eine gesonderte Rohrherstellung und eine anschließende Wiedererwärmung vollständig entfallen.

Ermöglicht wird die präzise Herstellung insbesondere dadurch, dass entgegen im Stand der Technik bekannter Press-Blas-Verfahren eine nach unten offene Pressform verwendet wird. Auf diese Weise werden die Pressdrücke begrenzt und auch ein Auspressen eines sehr dünnen Külbels ermöglicht.

Außerdem weist der so hergestellte Behälter keine oder nur sehr geringe Oberflächenverarmungen an leicht flüchtigen Elementen wie etwa Bor oder Natrium auf.

Erfindungsgemäß wird der Külbel nach dem Ausfahren des Pressstempels vor dem anschließenden Blasschritt im mittleren Bereich erwärmt, vorzugsweise auf eine Temperatur, bei der die Viskosität des Glases weniger als 10^{4,7} dPas beträgt, und ferner an mindestens einem seiner beiden Enden gekühlt, vorzugsweise auf eine Temperatur, bei der die Viskosität mehr 10¹⁰ dPas beträgt.

Durch diese Maßnahmen wird eine gute Blasformung im mittleren Abschnitt gewährleistet, während der Külbel an seinen beiden Enden, d.h. am Flansch und am Konus, infolge ausreichender Abkühlung nicht weiter verformt wird.

Der Behälter kann in verschiedenen von der pharmazeutischen Industrie geforderten Dimensionen hergestellt werden. Es handelt sich um einen an beiden Enden offenen Hohlkörper, der vorzugsweise zylindrisch ausgebildet ist, insbesondere als Spritzenkörner oder Karpule.

Insbesondere weist der Behälter einen zylindrischen Abschnitt mit einem Flansch an seinem einen Ende und mit einem Konus an seinem anderen Ende auf.

Im Rahmen dieser Anmeldung wird unter "Konus" ein Bereich verstanden, der einen geringeren Durchmesser als der mittlere, zylindrisch ausgeführte Abschnitt besitzt. Die Form des Konus ist vom jeweiligen Produkt abhängig. In der Regel verjüngt sich der Konus zu seinem Ende hin, kann dort aber auch Verdickungen aufweisen.

Der Behälter wird in der Regel mit einem durchgehenden Konuskanal in seinem Konus hergestellt.

Soweit der Konuskanal nicht bereits während des Pressvorgangs an seinem Ende eröffnet werden kann, so dass die Endform erreicht ist, wird der Konus nach dem Pressvorgang an seinem äußeren Ende angeschnitten, um den Konuskanal zu eröffnen.

Der Külbels wird während des Pressschrittes vorzugsweise im Wesentlichen in Form eines hohlen Kegelstumpfes hergestellt.

Vorzugsweise weist der Külbel im zylindrischen Abschnitt des Behälters eine vom Konus zum Flansch hin abnehmende Wandstärke auf.

Auf diese Weise kann in Verbindung mit dem nachfolgenden Blasschritt der zylindrische Abschnitt mit einer engen Dickentoleranz hergestellt werden.

Gemäß einer weiteren Ausgestaltung der Erfindung wird der Külbel in einem ersten Pressschritt mit einem größeren Pressstempel ohne Konuskanal vorgeformt, und mit einem zweiten Pressschritt mit einem zweiten Pressstempel wird ein Konuskanal ausgeformt.

Hierbei kann der zweite Pressschritt von der dem Flansch gegenüberliegenden Seite aus erfolgen.

Alternativ kann auch ein im Pressstempel geführter Dorn vorgesehen sein, durch den der Konuskanal durch Ausfahren nach dem Auspressen des zugeführten Glasvolumens erzeugt wird.

Durch diese Maßnahme ergibt sich eine einfache Ausbildung des Konuskanals.

Gemäß einer weiteren Ausgestaltung der Erfindung wird der Glastropfen aus einem Speiser portioniert und fällt unmittelbar in die Form. Zur Portionierung wird vorzugsweise hierbei ein scherenloser Nadelspeiser verwendet. Dadurch werden Abdrücke von der Schere vermieden.

In alternativer Ausführung der Erfindung wird der Glastropfen aus einem Speiser portioniert, fällt auf die Pressform und wird erst durch den Pressstempel in die Pressform ausgepresst.

Mit beiden Varianten lässt sich eine Herstellung mit der gewünschten Maßhaltigkeit gewährleisten.

Gemäß einer weiteren Verfahrensvariante der Erfindung wird in einem ersten Pressschritt mit einem vorzugsweise durchgehend konischen Pressstempel ein Külbel geformt und in einem anschließenden Schritt der Pressstempel durch einen Dorn mit der gewünschten Form des Konuskanals ersetzt, der Külbel rotierend angetrieben und durch Anlegen von Formrollen im Bereich des Konus ausgeformt.

Hierbei wird der Külbel vor dem Anlegen der Formrollen vorzugsweise im Konusbereich wieder erwärmt.

Durch diese Maßnahmen lässt sich eine sehr hohe Maßhaltigkeit des hergestellten Behälters insbesondere im Konusbereich gewährleisten.

Gemäß einer weiteren Ausgestaltung der Erfindung wird während des Pressschrittes ein Vakuum angelegt.

Durch diese Maßnahme wird der Pressvorgang unterstützt und ein Erreichen der gewünschten Külbelform und Toleranz gefördert.

Ein erfindungsgemäßer Behälter für pharmazeutische oder medizinische Anwendungen aus Borosilikatglas, vorzugsweise aus einem Typ I-Glas, weist einen zylindrischen Bereich auf, der eine Wanddickentoleranz von maximal ± 0,2 mm aufweist, wobei der Borgehalt des Behälters gegenüber seinem nominellen Wert an allen Oberflächen weniger als 60 %, vorzugsweise weniger als 40 %, besonders bevorzugt weniger als 20 % abfällt.

Durch die erfindungsgemäße Herstellung unmittelbar aus der Schmelze ohne Benutzung von vorgeformten Rohren besitzt der erfindungsgemäße Behälter eine homogenere Glaszusammensetzung aus Borosilikatglas über seinen gesamten Bereich.

Gleichermaßen fällt vorzugsweise der Natriumgehalt des Behälters gegen seinen nominellen Wert an allen Oberflächen weniger als 30 %, vorzugsweise weniger als 20 % ab.

Der Behälter kann die verschiedensten von der pharmazeutischen Industrie geforderten Abmessungen aufweisen und insbesondere an seinem einen Ende einen Flansch und an seinem anderen Ende einen Konus besitzen.

Der erfindungsgemäße Behälter, der unmittelbar aus der Schmelze hergestellt ist, kann erfindungsgemäß auch mit sehr geringen Dimensionen hergestellt sein, und eine Gesamtglasmasse von höchstens 15 g, insbesondere von höchstens 10 g, insbesondere von höchstens 5 g aufweisen.

In entsprechender Weise kann das Volumen des Behälters höchstens 15 ml betragen, insbesondere höchstens 10 ml, insbesondere höchstens 5 ml.

Ferner kann der Behälter eine Gesamthöhe und einen Gesamtdurchmesser aufweisen, wobei die Gesamthöhe mindestens das 1,5-fache des Gesamtdurchmessers betragen kann.

Schließlich kann der Behälter auch eine sehr geringe Wandstärke aufweisen, die < 1,5 mm ist, insbesondere kleiner < 1,3 mm, insbesondere < 1,1 mm.

Dennoch kann die Toleranz der Wanddicke ≤ ± 0,2 mm betragen oder sogar ≤ ± 0,1 mm sein.

Weitere Merkmale und Vorteile der Erfindung ergeben sich aus der nachfolgenden Beschreibung bevorzugter Ausführungsbeispiele unter Bezugnahme auf die Zeichnung. Es zeigen:
- Fig. 1 a) - d): verschiedene Phasen des Pressvorgangs zur Herstellung des Külbels unmittelbar aus der Schmelze mittels Pressen in eine nach unten offene Pressform;
- Fig. 2: eine schematische Darstellung eines an den Pressvorgang gemäß Fig. 1 anschließenden Schrittes zur Erwärmung des Külbels in seinem mittleren Bereich und zum gleichzeitigen Kühlen an seinen beiden Enden;
- Fig. 3 a), b): einen sich an den Zwischenschritt gemäß Fig. 2 anschließenden Blasschritt in der Ausgangsphase und in der Endphase, bei dem der vorgeformte Külbel in einer Blasform in die Endform des Behälters geblasen wird;
- Fig. 4 a) - d): verschiedene Phasen eines alternativen Pressschrittes mit Vorpressen mit einem ersten Pressstempels und Nachpressen mit einem zweiten Pressstempels, der erst die gewünschte Endform des Konuskanals erzeugt;
- Fig. 5a) - d): eine weitere Alternative des erfindungsgemäßen Pressvorgangs in verschiedenen Phasen, wobei der portionierte Glastropfen in Abwandlung zu der Ausführung gemäß Fig. 1 nicht unmittelbar in die Pressform fällt, sondern zunächst auf der Oberfläche der Pressform aufsitzt und erst durch den Pressstempel in die Pressform hineingepresst wird;
- Fig. 6a), b): eine weitere Variante der Erfindung mit einer dem Pressschritt nachgeschalteten Konusausformung bei rotierend angetriebenem Külbel und Anlegen von Formrollen zum Ausformen des Külbels im Konusbereich;
- Fig. 7a), b): eine weitere Variante des erfindungsgemäßen Pressverfahrens, bei dem der Konuskanal in einem auf den ersten Pressschritt folgenden zweiten Pressschritt durch einen Pressstempel von der Konusseite her ausgeformt wird; und
- Fi.g. 8: eine vergrößerte Darstellung eines geformten Külbels nach dem Pressschritt in seiner bevorzugten Ausgestaltung, wobei erkennbar ist, dass der Külbel nach dem Pressschritt in seinem mittleren Bereich, d.h. dem späteren zylindrischen Bereich des Behälters, eine vom Konus zum Flansch hin abnehmende Wandstärke aufweist.

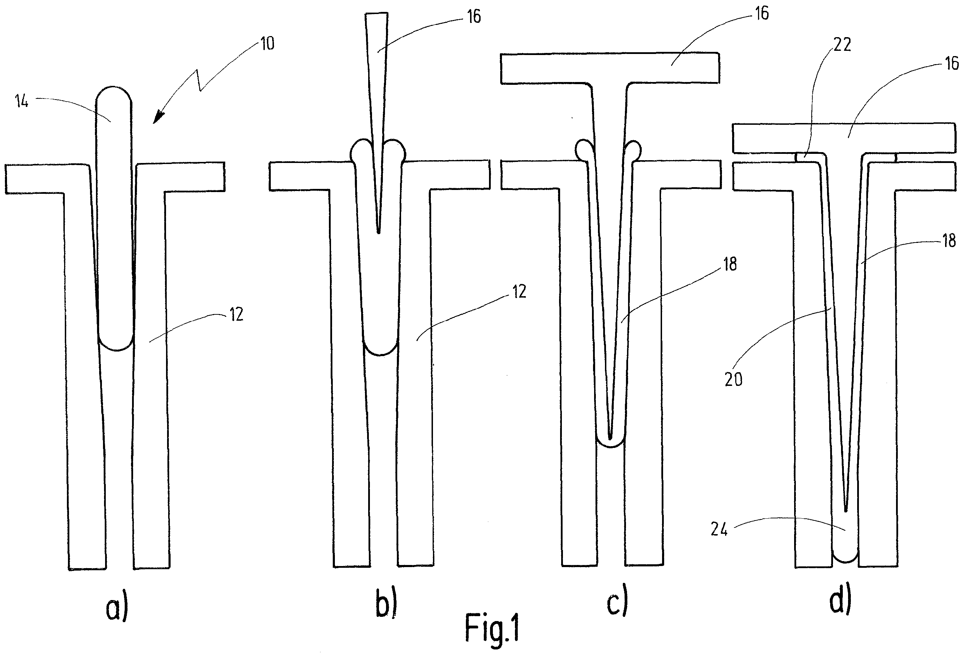

In Fig. 1 ist eine Pressvorrichtung zum Pressen eines Külbels 18 unmittelbar aus einem Tropfen aus der Schmelze dargestellt und insgesamt mit der Bezugsziffer 10 bezeichnet. Aus einem scherenlosen Nadelspeiser wird ein Tropfen 14 portioniert und fällt unmittelbar in eine Pressform 12 (Fig. 1a). Im nächsten Schritt gemäß Fig. 1b) fährt ein Pressstempel 16 nach unten und beginnt, den Tropfen 14 in eine Külbelform auszupressen. Gemäß Fig. 1c) ist der Külbel 18 schon zu einem großen Teil in die Pressform 12 eingepresst und besitzt gemäß Fig. 1d) bereits seine endgültige Form nach Beendigung der Pressstufe.

Im dargestellten Fall soll ein Behälter mit einem mittleren zylindrischen Abschnitt hergestellt werden, an dessen einem Ende ein Flansch angeformt ist und an dessen anderem Ende ein Konus mit einem geringeren Durchmesser als der zylindrische Abschnitt vorgesehen ist und der von einem durchgehenden Konuskanal durchsetzt ist.

Der Külbel 18, d.h. der vorgepresste Glaskörper, weist am Ende des Pressvorgang eine im Wesentlichen konische Form mit einem mittleren Abschnitt 20, einem Flansch 22 am einen Ende und dem Konus 24 am anderen Ende auf.

Der Konuskanal 40 ist am Ende des Pressvorgangs gemäß Fig. 1d) noch nicht vollständig ausgeformt.

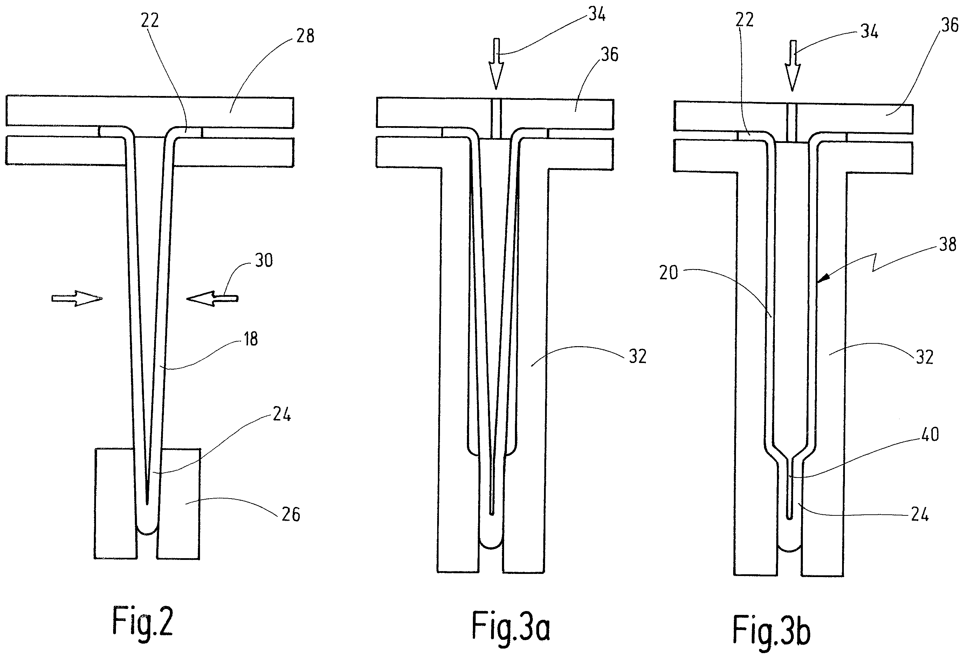

An den Pressvorgangs schließt sich das Ausformen des Külbels 18 in die endgültige Form des Behälters 38 durch einen Blasvorgang an, was in Fig. 3a), b) dargestellt ist.

Da der Behälter 38 sowohl im Bereich seines Flansches 22 als auch im Bereich des Konus 24 während des Blasschrittes jedoch nicht oder kaum verformt werden soll, wird vor dem Blasschritt gemäß Fig. 2 ein Zwischenschritt durchgeführt, bei dem der Külbel 18 in seinem mittleren Bereich mittels eines Brenners wieder erwärmt wird, während der Külbel 18 im Bereich seines Flansches 22 und im Bereich des Konus 24 jeweils durch einen Kühler 28 bzw. 26 gekühlt wird.

Nach diesem Zwischenschritt gemäß Fig. 2 wird der Külbel 18 in eine Blasform 32 überführt, deren Innenkontur der Endform des fertigen Behälters 38 gemäß Fig. 3 entspricht. Flanschseitig wird der Külbel 18 durch einen Gegenhalter 36 fixiert, der einen Durchlass zum Einblasen eines Fluids, z.B. Luft oder Stickstoff, aufweist, wie durch Ziffer 34 angedeutet. Während in Fig. 3a) der Beginn des Pressvorgangs dargestellt ist, ist in Fig. 3b) das Ende des Pressvorgangs gezeigt, bei dem der Külbel 18 nunmehr die Endform des Behälters 38 angenommen hat, mit einem mittleren zylindrischen Abschnitt 20, dem Flansch 22 am einen Ende und dem Konus 24 am anderen Ende. Der vom Inneren des zylindrischen Abschnitts 20 ausgehende Konuskanal 40 geht noch nicht bis nach außen durch und endet kurz vor dem äußeren Ende.

Um den Konuskanal 40 zu eröffnen, wird der Behälter 38 in einem späteren Schritt an seinem Konus 24 abgeschnitten, so dass der Konuskanal 40 nach außen mündet.

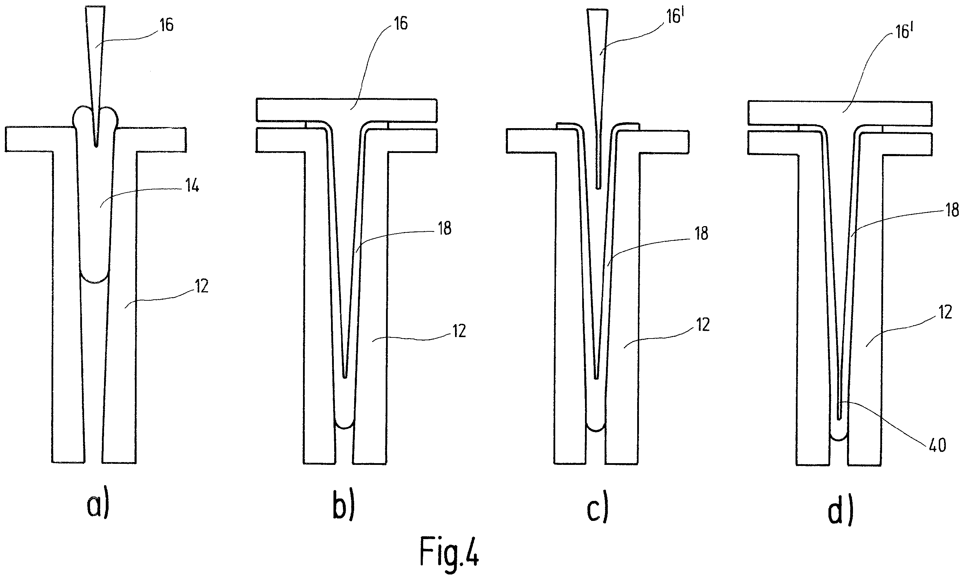

Verschiedene Verfahrensvarianten des zuvor anhand der Fig. 1 bis 3 beschriebenen Press-Blasverfahrens zur Herstellung des Behälters unmittelbar aus der Schmelze werden nachfolgend anhand der Fig. 4 bis 7 näher erläutert.

In Fig. 4 ist eine Variante des Pressschrittes dargestellt, bei der statt eines einzigen Pressstempels gemäß Fig. 1 nacheinander zwei unterschiedliche Pressstempel 16, 16' verwendet werden.

Mit dem ersten Pressstempel 16 wird der Külbels 18 in einer ausschließlich konischen Pressform 12 vorgepresst (vgl. Fig. 4a), b)), während in einem nachfolgenden zweiten Pressschritt der Külbel 18 mit einem zweiten Pressstempel 16' nachgepresst wird. Der erste Pressstempel 16 weist eine rein konische Form auf, während der zweite Pressstempel 16' an seinem unteren Ende die spätere Form des Konuskanal 40 als schlanken Fortsatz aufweist. So erlaubt es der zweite Pressstempel 16', den Konuskanal 40 bei kurzer Kontaktzeit im zweiten Pressschritt auszuformen, während im ersten Pressschritt gemäß der Fig. 4a), b) der Konuskanal 40 noch gar nicht ausgeformt ist. Durch dieses zweistufige Verfahren wird die thermische Belastung des Pressstempels reduziert und die Kontaktzeit verkürzt.

Im anschließenden Blasschritt bzw. Zwischenschritt erfolgt die Weiterverarbeitung des Külbels 18 wie zuvor anhand der Fig. 2 und 3 beschrieben.

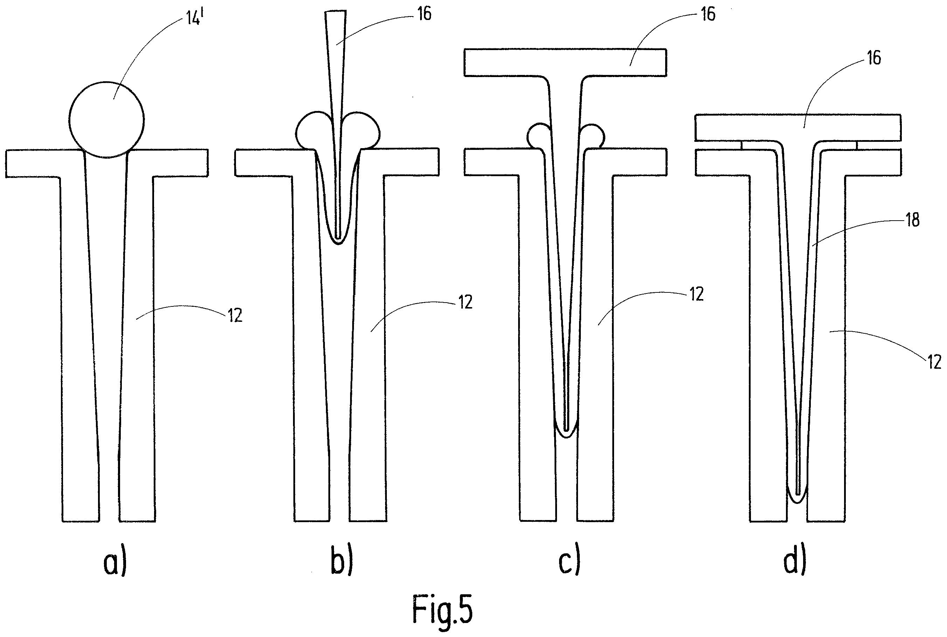

In Fig. 5 ist eine weitere Verfahrensvariante dargestellt. Hierbei fällt der aus dem Speiser portionierte Tropfen nicht unmittelbar in die nach unten offene Preßform 1.2, sondern gelangt zunächst auf das obere Ende der Pressform 12, wie durch den Tropfen 14' angedeutet ist (vgl. Fig. 5a)). Erst in den nachfolgenden Pressschritten gemäß Fig. 5b), c) und d) erfolgt die Ausformung des Külbels 18. Der Glastropfen 14' wird erst durch Niederfahren des 1'ressstempels 1.6 in die Pressform 12 ausgepresst und nimmt schließlich seine Endform gemäß Fig. 5d) an.

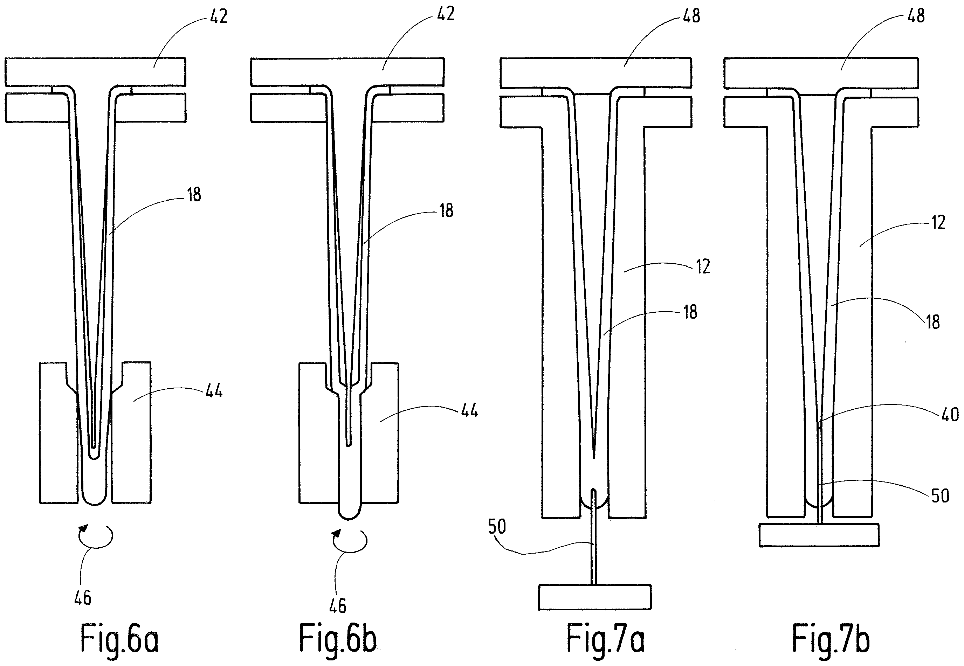

Eine weitere Verfahrensvariante ist in Fig. 6a), b) dargestellt. Hierbei wird der Külbel 18 zunächst durch Pressen hergestellt, wie zuvor anhand der Fig. 1a) bis 1d) erläutert wurde. Nach der Formung des Külbels 18 wird der Pressstempel 16 wieder herausgefahren und durch einen Dorn 42 ersetzt, dessen Form der gewünschten Endform des Külbels 18 entspricht, wobei insbesondere der Konuskanal 40 am unteren Ende angeformt ist. Der Külbel 18 wird nun in Rotation versetzt, wie durch den Pfeil 46 angedeutet ist, und es werden Formrollen 44 von außen nach und nach angedrückt, um den Konus in seine gewünschte Endform zu bringen, die in Fig. 6b) dargestellt ist.

Eine weitere Verfahrensvariante ist in Fig. 7a, b) dargestellt.

Hierbei wird der Külbel 18 zunächst, wie zuvor anhand von Fig. 1a) bis d) dargestellt, durch Pressen hergestellt. In einem anschließenden zweiten Pressschritt wird mittels eines zweiten Pressstempels 50 an der Konusseite der Konuskanal 40 eingestochen, so dass die endgültige Form des nach außen offenen Konuskanals 40 erreicht wird, ohne dass ein späteres Abschneiden des Behälters 38 zur Öffnung des Konuskanals 40 notwendig ist.

### Beispiel

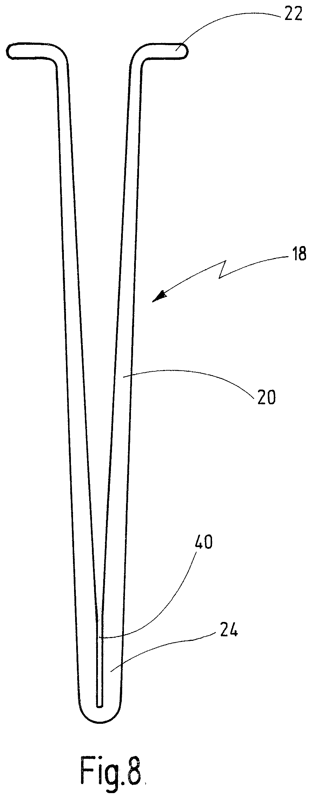

Aus dem Borosilikatglas FIOLAX®, das von der Anmelderin hergestellt und vertrieben wird, wird ein Spritzenkörper 38 hergestellt. Die charakteristischen Daten des verwendeten Glases FIOLAX® sind in Tab. 1 zusammengefasst. Es wird ein Spritzenkörper 38 mit einem Normvolumen von 1 ml, einem Durchmesser im zylindrischen Abschnitt von 8,15 mm und einer Gesamtlänge von 64 mm mit einer Wandstärke von 0,9 mm im Bereich des zylindrischen Abschnittes hergestellt. Nach der Portionierung eines Glastropfens 14 mittels eines scherenlosen Nadelspeisers fällt der Glastropfen 14 unmittelbar in die Pressform 12 gemäß Fig. 1a). Beim Pressschritt gemäß der Fig. 1b) bis d) wird nunmehr der Külbel 18 mit einer solchen Form ausgepresst, dass die Wandstärke im Bereich des mittleren Abschnittes 20 vom Konus 24 aus in Richtung zum Flansch 22 hin abnimmt, wie aus der vergrößerten Darstellung in Fig. 8 deutlich erkennbar ist.

Anschließend erfolgt gemäß Fig. 2 eine thermische Zwischenkonditionierung des Külbels 18 mittels lokaler Brennererwärmung an seinem mittleren Bereich 20 und Kontaktkühlung am Flansch 22 und am Konus 24. Hierbei wird der Külbel 18 am Flansch 22 und am Konus 24 auf eine Temperatur von < 650 °C, vorzugsweise auf etwa 600 °C, abgekühlt, während gleichzeitig im mittleren Abschnitt 20 eine Erwärmung auf mehr als 1050 °C, vorzugsweise auf annähernd 1200 °C erfolgt. Der Übergangsbereich zwischen der hohen Temperatur im mittleren Bereich 20 und der niedrigen Temperatur im Flansch 22 bzw. Konus 24 beträgt weniger als 10 mm.

Anschließend wird der so thermisch zwischenkonditionierte Külbel 18 in eine Blasform gemäß Fig. 3a) überführt und am Flanschende mittels eines Gegenhalters 36 verschlossen. Anschließend erfolgt die Blasformung mit einem Überdruck von etwa 0,5 bar bei einer Temperatur von mehr als 1050 °C im mittleren Bereich 20.

Der so hergestellte Spritzenkörper 38 liegt innerhalb seiner gewünschten geometrischen Spezifikation und weist insbesondere in seinem zylindrischen mittleren Bereich 20 eine Wandstärke von 0,9 mm ± 100 µm auf.

**Tab. 1**

| Daten von FIOLAX® | | | | | |
|---|---|---|---|---|---|
| Chemische Zusammensetzung (Hauptbestandteile in Gew.-%) | | | | | |
| SiO₂ | B₂O₃ | Al₂O₃ | Na₂O | CaO | |
| 75 | 10,5 | 5 | 7 | 1,5 | |

| Wasserbeständigkeit | | | | | |
|---|---|---|---|---|---|
| nach DIN ISO 719 | | | Klasse HGB 1 | | |

| Säurebeständigkeit | | | | | |
|---|---|---|---|---|---|
| nach DIN 12116 | | | Klasse S1 | | |

| Thermischer Ausdehnungskoeffizient | | | | | |
|---|---|---|---|---|---|
| α (20 °C) | | | 4,9 ·10⁻⁶/K | | |
| Transformationstemperatur T_{g} | | | 565 °C | | |

| Temperatur des Glases bei den Viskositäten η (in dPa·s) | | | | | |
|---|---|---|---|---|---|
| 10¹³ (Obere Kühltemperatur) | | | 565 °C | | |
| 10^{7,6} (Erweichungstemperatur) | | | 785 °C | | |
| 10⁴ (Verarbeitungstemperatur) | | | 1165 °C | | |
| Dichte bei 25 °C | | | 2.340 kg/m³ | | |

## Patentansprüche

1. Verfahren zum Herstellen eines Behälters in Form eines an beiden Enden offenen Hohlkörpers für pharmazeutische oder medizinische Anwendungen aus Glas, insbesondere in Form eines Spritzenkörpers vorzugsweise aus Borosilikatglas, bei dem der Behälter (38) durch ein Press-Blas-Verfahren hergestellt wird, bei dem der Behälter (38) zunächst in einem Pressschritt vorgeformt wird, indem ein portionierter Glastropfen (14) durch einen Pressstempel (16) in eine nach unten offene Pressform (12) gepresst wird, bei dem der so hergestellte Külbel (18) durch einen anschließenden Blasschritt endformnah ausgeformt wird und an seinem Ende eröffnet wird, wobei der Külbel (18) nach dem Ausfahren des Pressstempels (16) vor dem anschließenden Blasschritt im mittleren Abschnitt (20) erwärmt wird und an mindestens einem seiner beiden Enden gekühlt wird.

2. Verfahren nach Anspruch 1, bei dem der Behälter (38) mit einem mittleren, zylindrischen Abschnitt (20), mit einem Flansch (22) am einen Ende, mit einem Konus (24) an seinem anderen Ende und mit einem durchgehenden Konuskanal (40) hergestellt wird.

3. Verfahren nach einem der vorhergehenden Ansprüche, bei dem der Külbel (18) während des Pressschrittes in Form eines hohlen Kegelstumpfes hergestellt wird.

4. Verfahren nach einem der Ansprüche 2 bis 3, bei dem der Külbel (18) im zylindrischen Abschnitt (20) des Behälters (38) eine vom Konus (24) zum Flansch hin abnehmende Wandstärke aufweist.

5. Verfahren nach einem der vorhergehenden Ansprüche, bei dem der Külbel (18) nach dem Ausfahren des Pressstempels (16) vor dem anschließenden Blasschritt im mittleren Abschnitt (20) auf eine Temperatur erwärmt wird, bei der die Viskosität höchstens 10^{4,7} dPas beträgt.

6. Verfahren nach einem der vorhergehenden Ansprüche, bei dem der Külbel (18) nach dem Ausfahren des Pressstempels (16) an mindestens einem seiner beiden Enden auf eine Temperatur gekühlt wird, bei der die Viskosität mindestens 10¹⁰ dPas beträgt.

7. Verfahren nach einem der vorhergehenden Ansprüche, bei dem der Külbel (18) in einem ersten Pressschritt mit einem größeren Presstempel (16) ohne Konuskanal (40) vorgeformt wird und in einem zweiten Pressschritt mit einem zweiten Presstempel (16'; 48) ein Konuskanal (40) ausgeformt wird.

8. Verfahren nach Anspruch 7, bei dem der zweite Pressschritt von der dem Flansch (22) gegenüberliegenden Seite aus erfolgt.

9. Verfahren nach einem der vorhergehenden Ansprüche, bei dem der Glastropfen (14) aus einem Speiser portioniert wird und unmittelbar in die Form fällt.

10. Verfahren nach einem der Ansprüche 1 bis 8, bei dem der Glastropfen (14') aus einem Speiser portioniert wird, auf die Pressform (12) fällt und erst durch den Pressstempel (16) in die Pressform (12) ausgepresst wird.

11. Verfahren nach einem der Ansprüche 1 bis 6 oder 9 bis 10, bei dem in einem ersten Pressschritt mit einem vorzugsweise durchgehend konischen Pressstempel (16) ein Külbel (18) geformt wird, und in einem anschließenden Schritt der Pressstempel (16) durch einen Dorn (42) mit der Form des Konuskanals (40) ersetzt wird, der Külbel (18) rotierend angetrieben wird und durch Anlegen von Formrollen (44) im Bereich des Konus (24) ausgeformt wird.

12. Verfahren nach Anspruch 11, bei dem der Külbel (18) vor dem Anlegen der Formrollen (44) im Konusbereich (24) wieder erwärmt wird.

13. Verfahren nach einem der vorhergehenden Ansprüche, bei dem der vorgeformte Külbel (18) während des Blasschrittes rotierend angetrieben wird und vorzugsweise während des Pressschrittes ein Vakuum angelegt wird.

14. Verfahren nach einem der vorhergehenden Ansprüche, bei dem der Konuskanal (40) durch einen im Pressstempel geführten Dorn durch Ausfahren nach dem Auspressen des zugeführten Glasvolumens erzeugt wird.

15. Behälter in Form eines an beiden Enden offenen Hohlkörpers für pharmazeutische oder medizinische Anwendungen aus Borosilikatglas, insbesondere Spritzenkörper, vorzugsweise aus einem Typ I-Glas, mit einem zylindrischen Bereich (20), der eine Wanddickentoleranz von maximal ± 0,2 mm aufweist, wobei der Borgehalt des Behälters (38) gegenüber seinem nominellen Wert an allen Oberflächen weniger als 60 %, vorzugsweise weniger als 40 %, besonders bevorzugt weniger als 20 % abfällt.

## Claims

1. Process for producing of a container made from glass, in the form of a hollow body open on both ends for pharmaceutical and medical applications, in particular in the form of a syringe barrel preferably made from a borosilicate glass, wherein the container (38) is produced by a press-blow process, wherein the container (38) is initially preformed in a pressing step, by making a ram (16) press a dispensed glass drop (14) into a mold (12) that is open at its bottom, wherein the parison (18) so produced is given its final form by a subsequent blowing step and is opened at its end, wherein the parison (18) after removal of the ram (16) before the subsequent blowing step is heated in its medium portion (20) and is cooled at at least one of its ends.

2. The process according to claim 1, wherein the container is produced with a cylindrical medium portion (20) with a flange (22) at its one end, with a cone (24) on its other end and with a full-length cone channel (40).

3. The process according to any of the preceding claims, wherein the parison (18) is given the form of a hollow truncated cone in the pressing step.

4. The process according to any of claims 2 to 3, wherein the parison (18) has a wall thickness that tapers from the cone (24) toward the flange in the cylindrical portion (20) of the container (38).

5. The process according to any of the preceding claims, wherein the parison (18) after removal of the ram (16) and prior to the subsequent blowing step is heated in its medium portion (20) to a temperature at which the viscosity is less than 10^{4.7} dPas.

6. The process according to any of the preceding claims, wherein the parison (18) on at least one of its two ends after removal of the ram (16) is cooled to a temperature at which the viscosity is less than 10¹⁰ dPas.

7. The process according to any of the preceding claims, wherein the parison (18) is preformed without a cone channel (40) by a bigger ram (16) in a first pressing step and a cone channel (40) is then shaped using a second ram (16'; 48) in a second pressing step.

8. The process according to claim 7, wherein the second pressing step is carried out from the side opposite the flange (22).

9. The process according to any of the preceding claims, wherein the glass drop (14) is dispensed in portions by a feeder and drops directly into the mold.

10. The process according to any of claims 1 to 8, wherein the glass drop (14) is dispensed in portions by a feeder, drops onto the mold (12) and is only pressed into the press mold (12) by the ram (16).

11. The process according to any of claims 1 to 6 or 9 to 10, wherein a parison (18) is formed in a first pressing step, using a ram (16) that is conical preferably over its full length, the ram (16) is replaced in a subsequent step by a mandrel (42) having the shape of the cone channel (40), and the parison (18) is driven to rotate and is shaped by application of molding rolls (44) in the region of the cone (24).

12. The process according to claim 11, wherein the parison (18) is reheated once more in the area of the cone (24) before the molding rolls (44) are applied.

13. The process according to any of the preceding claims, wherein the preformed parison (18) is driven to rotate during the blowing step, and wherein preferably a vacuum is applied during the pressing step.

14. The process according to any of the preceding claims, wherein the cone channel (40) is produced by a stroke of a mandrel guided in the ram, after the glass volume supplied has been shaped in the pressing step.

15. Container for pharmaceutical and medical applications made from a borosilicate glass in the form of a hollow body that is open on both of its ends, in particular a syringe body, preferably made from a Type I glass, with a cylindrical portion (20) having a wall thickness tolerance of maximally ± 0.2 mm, where the boron content of the container (38), at all its surfaces, drops by less than 60 %, preferably less than 40 %, more preferably less than 20 % relative to its nominal value.

## Revendications

1. Procédé de fabrication d'un récipient en verre en forme de corps creux ouvert aux deux extrémités pour des applications pharmaceutiques ou médicales, en particulier sous la forme d'un corps de seringue de préférence en verre de borosilicate, dans lequel on fabrique le récipient (38) par un procédé pressé-soufflé, dans lequel le récipient (38) est d'abord préformé dans une étape de pressage en pressant une goutte de verre portionnée (14) au moyen d'un poinçon de presse (16) dans un moule de pressage (12) ouvert vers le bas, dans lequel l'ébauche ainsi fabriquée (18) est façonnée à sa forme presque définitive par une étape de soufflage qui suit et elle est ouverte à son extrémité, dans lequel l'ébauche (18) est chauffée dans sa région moyenne (20) après le retrait du poinçon de presse (16) et avant l'étape de soufflage qui suit et elle est refroidie à au moins une de ses deux extrémités.

2. Procédé selon la revendication 1, dans lequel le récipient (38) est fabriqué avec une partie moyenne cylindrique (20), avec une bride (22) à une extrémité, avec un cône (24) à son autre extrémité et avec un canal de cône continu (40).

3. Procédé selon l'une quelconque des revendications précédentes, dans lequel l'ébauche (18) est fabriquée à la forme d'un tronc de cône creux pendant l'étape de pressage.

4. Procédé selon l'une des revendications 2 à 3, dans lequel l'ébauche (18) présente dans la partie cylindrique (20) du récipient (38) une épaisseur de paroi décroissante du cône (24) à la bride.

5. Procédé selon l'une quelconque des revendications précédentes, dans lequel l'ébauche (18) est chauffée dans la partie moyenne (20), après le retrait du poinçon de presse (16) et avant l'étape de soufflage qui suit, à une température à laquelle la viscosité vaut au moins 10^{4,7} dPas.

6. Procédé selon l'une quelconque des revendications précédentes, dans lequel l'ébauche (18) est refroidie à au moins une de ses deux extrémités, après le retrait du poinçon de presse (16), à une température à laquelle la viscosité vaut au moins 10¹⁰ dPas.

7. Procédé selon l'une quelconque des revendications précédentes, dans lequel l'ébauche (18) est préformée sans canal de cône (40) dans une première étape de pressage avec un plus grand poinçon de presse (16) et un canal de cône (40) est finalement formé dans une deuxième étape de pressage avec un deuxième poinçon de presse (16'; 48).

8. Procédé selon la revendication 7, dans lequel la deuxième étape de pressage est effectuée à partir du côté opposé à la bride (22).

9. Procédé selon l'une quelconque des revendications précédentes, dans lequel la goutte de verre (14) est portionnée à partir d'un feeder et tombe directement dans le moule.

10. Procédé selon l'une quelconque des revendications 1 à 8, dans lequel la goutte de verre (14') est portionnée à partir d'un feeder, tombe sur le moule de presse (12) et n'est pressée dans le moule de presse (12) que par le poinçon de presse (16).

11. Procédé selon l'une quelconque des revendications 1 à 6 ou 9 à 10, dans lequel une ébauche (18) est formée dans une première étape de pressage avec un poinçon de presse (16) de préférence conique continu, et le poinçon de presse (16) est dans une étape suivante remplacé par une épine (42) ayant la forme du canal conique (40), l'ébauche (18) est entraînée en rotation et est façonnée dans la région du cône (24) par l'application de galets de formage (44).

12. Procédé selon la revendication 11, dans lequel l'ébauche (18) est de nouveau chauffée avant l'application des galets de formage (44) dans la région du cône (24).

13. Procédé selon l'une quelconque des revendications précédentes, dans lequel l'ébauche préformée (18) est entraînée en rotation pendant l'étape de soufflage et un vide est de préférence appliqué pendant l'étape de pressage.

14. Procédé selon l'une quelconque des revendications précédentes, dans lequel le canal de cône (40) est produit par une épine guidée dans le poinçon de presse par retrait après le pressage du volume de verre fourni.

15. Récipient en verre de borosilicate en forme de corps creux ouvert aux deux extrémités pour des applications pharmaceutiques ou médicales, en particulier corps de seringue, de préférence en un verre de type I, avec une région cylindrique (20), qui présente une tolérance sur l'épaisseur de paroi de ± 0,2 mm au maximum, dans lequel la teneur en bore du récipient (38) chute par rapport à sa valeur nominale à moins de 60 %, de préférence à moins de 40 %, et de préférence encore à moins de 20 % sur toutes les surfaces.
